# EUROPÄISCHE PATENTSCHRIFT

(11) **EP 0 432 743 B1**
(45) Veröffentlichungstag und Bekanntmachung des Hinweises auf die Patenterteilung: **19.07.1995**
(21) Anmeldenummer: 90123866.7
(22) Anmeldetag: 11.12.1990
(51) Int. Cl.: A61B 17/08

(54) **Wundklammer, insbesondere chirurgischer Mikroclip**
Wound clip, particularly surgical microclip
Clip pour plaie, en particulier microclip chirurgical

(30) Priorität: 12.12.1989 DE 8914582 U
(43) Veröffentlichungstag der Anmeldung: 19.06.1991
(73) Patentinhaber: Hubbes, Hilmar, D-32791 Lage (DE)
(72) Erfinder: Hubbes, Hilmar, D-32791 Lage (DE)
(74) Vertreter: Thielking, Bodo, Dipl.-Ing.

(56) Entgegenhaltungen:
- DE-A- 617 447
- FR-A- 370 297
- US-A- 4 217 902

## Beschreibung

Die Erfindung betrifft eine Wundklammer, insbesondere einen chirurgischen Mikroclip, mit zwei einander gegenüberliegenden Flanschbereichen aus Metallblech, welche an einem Ende in Richtung aufeinander zu weisende, aus dem Metallblech der Flansche herausragende Spitzen und an dem anderen Ende gegen Federkraft zusammendrückbare Griffbereiche aufweist.

Bei einer bekannten Wundklammer dieser Art sind zwei über eine gemeinsame Achse verbundene Blechteile vorgesehen. Die Achse wird von einer Wendelfeder umschlossen. Die Wendelfeder drückt die Blechteile in Richtung ihrer Spitzen zusammen. Die Federkraft wird für ein Öffnen der Wundklammer durch das Zusammendrücken der Griffbereiche überwunden.

Die bekannte Wundklammer ist vergleichsweise kompliziert. Sie besteht aus mehreren Einzelteilen und ist vergleichsweise teuer. Es ist sehr schwierig, diese Klammer zu reinigen, um sie mehrfach gebrauchen zu können.

Es ist ferner eine einstückige Wundklammer bekannt (US-A-4,217,902), die zwei einander gegenüberliegende, auswärts gebogene Flansche aufweist, die an ihrem unteren Ende einander entgegengerichtete Spitzen aufweist. Die Flansche sind an ihren, den Spitzen abwandten Enden mittels eines Biegebereichs verbunden, der in Richtung auf die Spitzen weist. Eine solche, nur mit Zange verwendbare Wundklammer kann nicht mit der gewünschten Feinfühligkeit gesetzt werden, wie dies bei Wundklammern mit Handgriffbereichen möglich ist und gefordert wird.

Ausgehend von diesem Stand der Technik liegt der Erfindung die Aufgabe zugrunde, eine Wundklammer der als bekannt vorausgesetzten Art so auszubilden, daß sie einfach und preisgünstig herstellbar und vergleichsweise leicht zu reinigen ist. Die Lösung dieser Aufgabe erfolgt mit den Merkmalen des Kennzeichnungsteils von Anspruch 1.

Die einteilige Ausbildung der Wundklammer läßt eine einfache und preisgünstige Fertigung zu. Darüber hinaus läßt sich eine solche erfindungsgemäße Wundklammer vergleichsweise einfach reinigen. Die Wölbung des Verbindungsflansches in Richtung auf die freien Enden der Griffbereiche hat einerseits zur Folge, daß der Verbindungsflansch nicht in eine störende Nähe zur Wunde gelangen kann und andererseits erlaubt sie, der einteiligen Wundklammer die gewünschten Elastizitätseigenschaft zu geben, wie sie beim Stand der Technik bisher nur durch Vorsehen einer zusätzlichen Wendelfeder erzielbar sind.

Es hat sich als zweckmäßig erwiesen, die Wundklammer aus Federstahlblech herzustellen.

Bei einer weiteren bevorzugten Ausführungsform der Erfindung sind die die Spitzen aufweisenden, einander gegenüberliegenden Flanschbereiche jeweils doppelt vorgesehen und durch eine sich in der Verlängerung des Verbindungsflansches erstreckende Aussparung voneinander getrennt.

Bevorzugte weitere Ausführungsformen der Erfindung sind in den weiteren Unteransprüchen beschrieben.

Nachstehend wird eine bevorzugte Ausführungsform der Erfindung anhand der Zeichnung im einzelnen beschrieben.

Es zeigen:
- Figur 1: eine perspektivische Darstellung einer ersten Ausführungsform der Wundklammer,
- Figur 2: eine perspektivische Ansicht einer im Aufbau gleichen, jedoch geringfügig anders geformten Ausführungsform der Wundklammer,
- Figur 3: eine Ansicht des flachen, später zur Wundklammer verformten einteiligen Stanzteils,
- Figur 4: eine Stirnansicht der Wundklammer im eingesetzten Zustand.

Die Wundklammer besteht aus zwei Seiten, welche über einen einteilig an beiden Seiten angeformten Verbindungsflansch 3 miteinander verbunden sind.

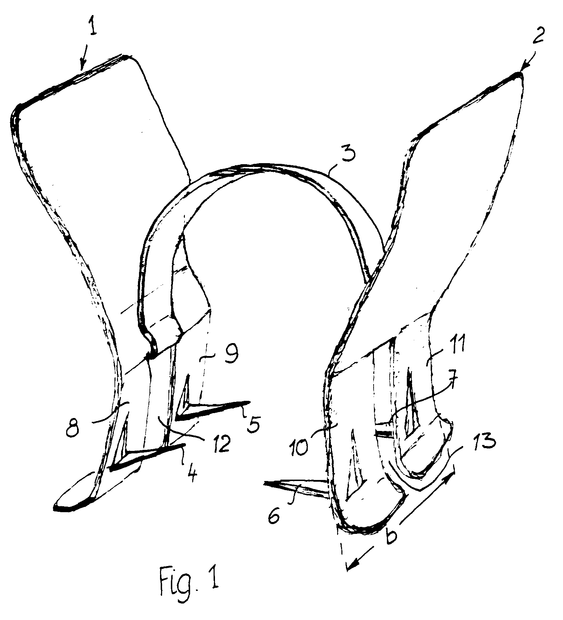

Das gemäß Figur 1 linke Seitenteil besitzt einen Griffbereich 1,an den sich zwei Flanschbereiche 8 und 9 anschliessen, welche sich zur Klammermitte erstreckende Spitzen 4 und 5 aufweisen.

Die gemäß Figur 1 rechte Seite besitzt einen Griffbereich 2, an den sich unten die beiden Flanschbereiche 10 und 11 mit Spitzen 6 und 7 anschließen.

Die beiden Flanschbereiche 8 und 9 einerseits sowie 10 und 11 andererseits sind durch Aussparungen 12 und 13 voneinander getrennt. Die Breite der Aussparung entspricht der Breite des Flanschbreichs 3. Die Breite b der Wundklammer ist über die gesamte Höhe annähernd gleich.

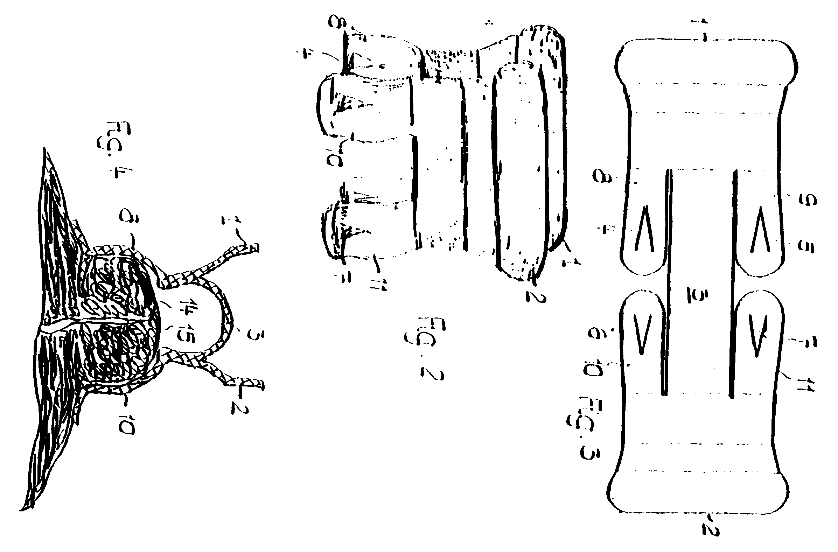

In Figur 3 ist das die Wundklammer gemäß Figuren 1 oder 2 bildende flachliegende Blechstanzteil gezeigt.

Figur 4 zeigt eine Stirnansicht der Wundklammer im eingesetzten Zustand. Es werden die beiden Wundränder 14 und 15 miteinander verklammert.

## Patentansprüche

1. Wundklammer, insbesondere chirurgischer Mikroclip, mit zwei einander gegenüberliegenden Flanschbereichen (8, 9; 10, 11) aus Metallblech, welche an einem Ende in Richtung aufeinander zu weisende,aus dem Metallblech der Flansche herausragende Spitzen (4, 5; 6, 7) und an dem anderen Ende gegen Federkraft zusammendrückbare Griffbereiche (1; 2) aufweist,
dadurch gekennzeichnet,
daß die Wundklammer einstückig ausgebildet ist und einen sich zwischen den Griffbereichen (1; 2) erstreckenden, in Richtung auf die freien Enden der Griffbereiche (1; 2) gewölbten, federnd elastischen Verbindungsflansch (3) aufweist.

2. Wundklammer nach Anspruch 1,
dadurch gekennzeichnet,
daß sie aus Federstahlblech besteht.

3. Wundklammer nach Anspruch 1 oder 2,
dadurch gekennzeichnet,
daß die die Spitzen (4; 5 und 6; 7) aufweisenden, einander gegenüberliegenden Flanschbereiche (8 und 9; 10 und 11) jeweils doppelt vorgesehen und durch eine sich in der Verlängerung des Verbindungsflansches (3) erstreckende Aussparung voneinander getrennt sind.

4. Wundklammer nach einem oder mehreren der Ansprüche 1 bis 3,
dadurch gekennzeichnet,
daß die die Spitzen (4; 5; 6; 7) aufweisenden Flanschbereiche (8; 9; 10; 11) an ihren freien Enden abgerundet ausgebildet sind.

5. Wundklammer nach einem oder mehreren der Ansprüche 1 bis 4,
dadurch gekennzeichnet,
daß sich die Griffbereiche (1; 2) über die gesamte Breite (b) der Wundklammer erstrecken.

6. Wundklammer nach einem oder mehreren der Ansprüche 1 bis 5,
dadurch gekennzeichnet,
daß der Verbindungsflansch (3) am Übergang zu den durch Flanschbereiche (8; 9; 10; 11) und Griffbereiche (1; 2) gebildeten Seiten zunächst mit einem engen Radius zur Klammermitte und danach in einem geringen Abstand von den Seiten verläuft, bevor er eine mittlere gemeinsame Wölbung bildet.

7. Wundklammer nach einem oder mehreren der Ansprüche 1 bis 6,
dadurch gekennzeichnet,
daß sie ein verformtes Stanzteil aus dünnwandigem Metallblech ist.

## Claims

1. Wound clamp, more particularly surgical micro-clamp, with two opposing flange areas (8,9;10,11), of metal plate which has at one end points (4,5;6,7) projecting out of the metal plate of the flanges and pointing towards each other, and at the other end has grip areas (1;2) which can be pressed together against spring force,
characterised in that the wound clamp is made in one piece and has a resiliently elastic connecting flange (3) which extends between the grip areas (1;2) and is curved towards the free ends of the grip areas (1;2).

2. Wound clamp according to claim 1, characterised in that it is made from sprung steel plate.

3. Wound clamp according to claim 1 or 2 characterised in that the flange areas (8 and 9; 10 and 11) facing opposite each other and having the points (4; 5 and 6;7) are each provided doubled and are separated from each other by a recess extending in the extension of the connecting flange (3).

4. Wound clamp according to one or more of claims 1 to 3 characterised in that the flange areas (8;9; 10; 11) having the points (4;5;6;7) are rounded at their free ends.

5. Wound clamp according to one or more of claims 1 to 4 characterised in that the grip areas (1;2) extend over the entire width (b) of the wound clamp.

6. Wound clamp according to one or more of claims 1 to 5 characterised in that the connecting flange (3) runs at the transition to the sides formed by the flange areas (8;9;10;11) and grip areas (1;2) first with a narrow radius to the clamp centre and then at a slight distance from the sides before it forms a central common curvature.

7. Wound clamp according to one or more of claims 1 to 6 characterised in that it is a deformed punched part from thin-walled metal plate.

## Revendications

1. Agrafe, tout particulièrement microclip chirurgical, avec deux zones de brides en tôle d'acier se faisant face (8, 9; 10, 11), présentant, à une extrémité, des pointes (4, 5; 6, 7) dirigées les unes vers les autres et faisant saillie hors de la tôle d'acier, et, à l'autre extrémité, des zones à griffes (1; 2) comprimables contre un effet de ressort,
caractérisé en ce que
l'agrafe est conçue d'une seule pièce et présente une bride de raccordement (3) élastique, laquelle s'étend entre les zones à griffes (1; 2) et est voûtée en direction des extrémités libres de celles-ci.

2. Agrafe selon la revendication 1,
caractérisée en ce
qu'elle est exécutée en tôle d'acier à ressort.

3. Agrafe selon la revendication 1 ou 2,
caractérisée en ce que
les zones de bride (8, 9; 10, 11) équipées des pointes (4; 5 et 6; 7) et se faisant face sont prévues en double et sont séparées par un évidement s'étendant dans le prolongement de la bride de raccordement (3).

4. Agrafe selon l'une ou plusieurs des revendications 1 à 3,
caractérisée en ce que
les tones de bride (8; 9; 10; 11) présentant les pointes (4; 5; 6; 7) sont arrondies à leurs extrémités libres.

5. Agrafe selon l'une ou plusieurs de revendications 1 à 4,
caractérisée en ce que
les zones à griffes (1; 2) s'étendent sur toute la longueur (b) de l'agrafe.

6. Agrafe selon l'une ou plusieurs de revendications 1 à 5,
caractérisée en ce que
la bride de raccordement (3), à la transition aux cûtés formés par les zones de bride (8; 9; 10; 11) et les zones à griffes (1; 2), s'étend, tout d'abord, avec un rayon plus étroit, vers le milieu de l'agrafe, puis, à une faible distance des côtés avant de former une voûte commune, médiane.

7. Agrafe selon l'une ou plusieurs des revendications 1 à 6,
caractérisée en ce
qu'elle consiste en une pièce estampée, déformée en tôle métallique fine.
